# EUROPEAN PATENT APPLICATION

(11) **EP 4 686 455 A1**
(43) Date of publication of application: **04.02.2026**
(21) Application number: 25193296.8
(22) Date of filing: 31.07.2025
(51) Int. Cl.: A61B 17/80

(54) **MODULAR PLATE TIP FOR PROPHYLACTIC NECK FIXATION**

(30) Priority: 02.08.2024 US 202463678648 P
(71) Applicant: Stryker European Operations Limited, Carrigtwohill, Co. Cork T45 HX08 (IE)
(72) Inventor: ZANDER, Nils, 24340 ECKERNFÖRDE (DE); WIELAND, Manfred, 24146 KIEL (DE); KLUEVER, Hendrik, 24232 SCHOENKIRCHEN (DE)
(74) Representative: Regimbeau

(57) **Abstract**

An extension plate, attachable to a bone plate and a bone, includes a body having a connection portion connectable to a bone plate and an anchoring portion attachable to a bone. The connection portion has a top surface configured to face away from a bone and a bottom surface configured to face toward the bone. The bottom surface of the connection portion has a first shelf extending substantially perpendicular from the bottom surface at a first side of the bottom surface and a second shelf extending substantially perpendicular from the bottom surface at a second side of the bottom surface. The second shelf includes a rail extending toward the first shelf, such that the second shelf defines an undercut between the rail and the bottom surface.

## Description

### CROSS-REFERENCE TO RELATED APPLICATION

The present application claims priority to U.S. Application No. 63/678,648 filed August 2, 2024, the disclosure of which is incorporated herein by reference.

### BACKGROUND OF THE INVENTION

The present disclosure relates to a modular plate tip for prophylactic neck fixation for use in the treatment of a metaphyseal fracture.

There are many instances in the treatment of complex bone fractures of using intramedullary nails, locking plates, or a nail-plate combination (NPC). Specifically, NPCs have been utilized to treat fractures within the distal femoral metaphyseal region of patients. However, abrupt changes of local construct stiffness may lead to increased stress points proximal to ends of the intramedullary nail and locking plate, particularly when those ends are close to one another within the bone. These increased stress point may result in an increased risk of fractures proximal to this region of intersection of the NPC. Additionally, geriatric patients often suffer from additional increased risks of fractures due to increased incidence/frequency of post-operative accidents and the potential need for weight bearing restrictions.

In order to protect patients from this increased risk of fractures, plate-based prophylactic fixation or stabilization of the femoral neck is commonly utilized. Prophylactic stabilization of the femoral neck requires an extended plate length to follow the shape of the vastus ridge, along with a lateral bend and an anterior external twist to compensate for the anteversion of the femoral neck. Therefore, pre-shaped plates are economically infeasible for prophylactic stabilization of the femoral neck, due to the high degree of anatomical variations of patients and the need to optimize plate fit to the bone for treatment of the fracture. Additionally, plates for prophylactic stabilization of the femoral neck currently offer limited opportunities for intraoperative design and modification, due to the need to provide maximum strength and stability to the bone fracture. Thus, improvements for plate-based prophylactic stabilization of the femoral neck are needed.

### BRIEF SUMMARY OF THE INVENTION

A first aspect of the present invention is an extension plate attachable to a bone plate and a bone, the extension plate including: a body having a connection portion connectable to a bone plate and an anchoring portion attachable to a bone; the connection portion having a top surface configured to face away from a bone and a bottom surface configured to face toward the bone; the bottom surface of the connection portion having a first shelf extending substantially perpendicular from the bottom surface at a first side of the bottom surface and a second shelf extending substantially perpendicular from the bottom surface at a second side of the bottom surface; wherein the second shelf includes a rail extending toward the first shelf, such that the second shelf defines an undercut between the rail and the bottom surface.

In accordance with other embodiments of the first aspect, the connection portion may define an aperture extending from the top surface to the bottom surface between the first and second sides in which a locking element can be disposed. The extension plate may further include a locking element having proximal and distal ends and a middle portion therebetween, the proximal and distal ends each having a diameter larger than a minimum diameter of the aperture, and the middle portion having a diameter smaller than the minimum diameter of the aperture. The locking element may include a locking screw and a locking collar, the proximal end of the locking element being a head of the locking screw, and the distal end of the locking element being the locking collar. The locking screw may be configured to be threadedly inserted into a passage of the locking collar, whereby rotation of the locking screw with respect to the locking collar changes a length of the locking element. The bottom surface of the connection portion may define a recess coaxial with the aperture, the recess having a non-circular perimeter. An end of the locking collar may have a non-circular outer perimeter dimensioned to be disposed within the recess such that the locking collar is rotationally fixed with respect to the recess.

Continuing with other embodiments of the first aspect, the anchoring portion may have a top surface configured to face away from the bone and a bottom surface configured to face toward the bone, and an aperture extending from the top surface of the anchoring portion to the bottom surface of the anchoring portion in which a fastener can be disposed. A central axis of the aperture in the connection portion and a central axis of the aperture in the anchoring portion may be non-parallel. Along an axis extending from the connecting portion to the anchoring portion, the top surface of the connecting portion may extend along a substantially straight axis, and the top surface of the anchoring portion may extend along a substantially curved axis. The bottom surface of the connection portion may further include an end shelf extending substantially perpendicular from the bottom surface and connecting ends of the first and second shelves. The bottom surface of the connection portion may define ridges. The ridges may be configured to mate with a detent on a bone plate.

A second aspect of the present invention is a bone plate system including: a bone plate having an anchoring section and a distal end extending from the anchoring section, wherein the anchoring section defines an aperture in which a fastener can be disposed to anchor the bone plate to a bone; and the extension plate of the first aspect, wherein the connection portion of the extension plate is configured to be secured to the distal end of the bone plate, such that the anchoring portion of the extension plate can be secured to the bone so that the bone plate system has a length longer than a length of the bone plate.

In accordance with other embodiments of the second aspect, the distal end of the bone plate may extend away from the anchoring section of the bone plate along a plate axis, the distal end of the bone plate may have first and second side edges extending parallel to the plate axis, the distal end of the bone plate may have a top surface configured to face away from a bone and a bottom surface configured to face toward the bone, wherein an outer surface of the first edge may extend from the top surface to the bottom surface along a first edge axis, and an outer surface of the second edge may extend from the top surface to the bottom surface along a second edge axis that is angled with respect to the first edge axis. The top surface of the distal end of the bone plate may include a detent protruding upward and away from the top surface. The detent may be positioned to engage with the ridges on the bottom surface of the connection portion of the extension plate. The outer surface of the second edge may extend from the top surface to the bottom surface towards the first edge.

Continuing with other embodiments with the second aspect, the distal end of the bone plate may extend away from the anchoring section of the bone plate along a plate axis, the distal end of the bone plate may have first and second side edges extending parallel to the plate axis, the distal end of the bone plate may have a top surface configured to face away from a bone and a bottom surface configured to face toward the bone, wherein the distal end of the bone plate may define an opening extending parallel to the plate axis in between the first and second edges. A first width at a proximal portion of the opening may be greater than a second width at a distal portion of the opening. The proximal portion of the opening may be substantially circular in shape and the distal end of the opening may be substantially obround in shape. The distal portion of the opening may include a rim extending inwardly toward a center of the opening.

In the extension plate, the rail may be a protrusion disposed at the end of an arm. The arm may be a cantilever beam defined by a cutout in the second shelf. The arm may be configured to flex toward and away from the first shelf. A length of the protrusion may be shorter than a length of the second shelf. The first shelf may include a rail extending toward the second shelf, such that the first shelf defines an undercut between the rail of the first shelf and the bottom surface. The rail of the first shelf may be a protrusion disposed at the end of an arm. The arm of the first shelf may be a cantilever beam defined by a cutout in the first shelf. The arm of the first shelf may be configured to flex toward and away from the second shelf. A length of the protrusion of the first shelf may be shorter than a length of the first shelf. The first shelf may be a mirror image of the second shelf. The extension plate may further include a locking element. The locking element may be a locking screw.

Another aspect of the present invention is a bone plate system including a bone plate having an anchoring section and a distal end extending from the anchoring section, wherein the anchoring section defines an aperture in which a fastener can be disposed to anchor the bone plate to a bone, and the extension plate of any of the foregoing aspects. The connection portion of the extension plate is configured to be secured to the distal end of the bone plate, such that the anchoring portion of the extension plate can be secured to the bone so that the bone plate system has a length longer than a length of the bone plate.

In other embodiments of this aspect, The distal end of the bone plate may extend away from the anchoring section of the bone plate along a plate axis, the distal end of the bone plate having first and second side edges extending parallel to the plate axis, the distal end of the bone plate having a top surface configured to face away from a bone and a bottom surface configured to face toward the bone, wherein an outer surface of the first edge extends from the bottom surface to a ledge at the top surface along a first edge axis, and an outer surface of the second edge extends from the bottom surface to a ledge at the top surface along a second edge axis. The outer surfaces of the first and second edges may each include ridges. The ridges may be configured to engage the protrusion disposed at the end of the arm.

A third aspect of the present invention is a method for extending a length of a bone plate system along a bone, the method including: anchoring a proximal end of a bone plate to a first portion of the bone, the bone plate extending along a plate axis; fixing a connection portion of an extension plate to a distal end of the bone plate, wherein the connection portion is tilted at a first angle about the plate axis to engage the distal end and then rotated through a second angle about the plate axis to be secured to the distal end; sliding the extension plate along the plate axis in a first direction away from the bone plate; locking the connection portion to the distal end; and anchoring a distal end of the extension plate to a second portion of the bone.

The connection portion may define a first aperture extending therethough from a top surface to a bottom surface of the connection portion, and the distal end may define a second aperture extending therethough from a top surface to a bottom surface of the distal end, and the locking step may further include securing a locking screw disposed through the first and second apertures to fix the connection portion to the distal end.

In other embodiments of the third aspect, the method may further include the step of sliding the extension plate along the plate axis in a second direction towards the bone plate. Anchoring the distal end of the extension plate may further include moving the distal end of the extension plate away from the plate axis. Anchoring the distal end of the extension plate may further include rotating the distal end of the extension plate about an axis parallel to the plate axis. Anchoring the distal end of the extension plate may further include rotating the distal end of the extension plate about an axis traverse to the plate axis.

Another aspect of the present invention is a method for extending a length of the bone plate system above along a bone, the method including anchoring the proximal end of the bone plate to a first portion of the bone, fixing the connection portion of the extension plate to the distal end of the bone plate by sliding the extension plate over the distal end of the bone plate and towards the bone plate along the plate axis, locking the connection portion to the distal end, and anchoring the distal end of the extension plate to a second portion of the bone. The step of fixing may include passing the protrusions of the first and second shelves over ridges of outer surfaces of the first and second edges of the distal end of the bone plate, and allowing the protrusions to engage specific ridges to temporarily fix the extension plate to the bone plate. The step of fixing may include passing the ridges on the bottom surface of the connection portion of the extension plate over the detent on the top surface of the distal end of the bone plate, and allowing the detent to engage a specific ridge to temporarily fix the extension plate to the bone plate. The connection portion may define a first aperture extending therethough from a top surface to a bottom surface of the connection portion, and the distal end may define a second aperture extending therethough from a top surface to a bottom surface of the distal end, the locking step may further include securing a locking screw disposed through the first and second apertures to fix the connection portion to the distal end.

### BRIEF DESCRIPTION OF THE DRAWINGS

The drawings described herein are for illustrative purposes only of the selected embodiments and are not all possible implementations and thus are not intended to limit the scope of the present disclosure.
FIGS. 1A-1B are perspective views of an extension plate of a bone plate system according to an embodiment of the present disclosure.
FIGS. 2A-2C are perspective views of a distal end of a bone plate of the bone plate system.
FIGS. 3A-3B are cross-sectional views of the bone plate system.
FIGS. 4A-4E are perspective views of the extension plate shown in FIGS. 1A-1B and the bone plate shown in FIGS. 2A-2C.
FIGS. 5A-5C are perspective views of a bone plate system installed on a bone according to another embodiment of the present disclosure.
FIG. 6A is a bottom perspective view of an extension plate in connection with a bone plate system according to another embodiment of the present disclosure.
FIG. 6B is a perspective view of a distal end of a bone plate of the bone plate system in accordance with the embodiment of FIG. 6A.
FIG. 6C is a perspective view of the extension plate of FIG. 6A with the bone plate of FIG. 6B installed on a bone.
FIGS. 7A and 7B are perspective views of left and right prophylactic neck attachments, respectively, in connection with a bone plate system according to another embodiment of the present disclosure.
FIG. 7C is a perspective view of a connection screw for use with the neck attachments of FIGS. 7A and 7B.
FIG. 8A is a bottom perspective view of an extension plate in connection with a bone plate system according to another embodiment of the present disclosure.
FIGS. 8B and 8C are perspective views of a distal end of a bone plate of the bone plate system in accordance with the embodiment of FIG. 8A.

### DETAILED DESCRIPTION

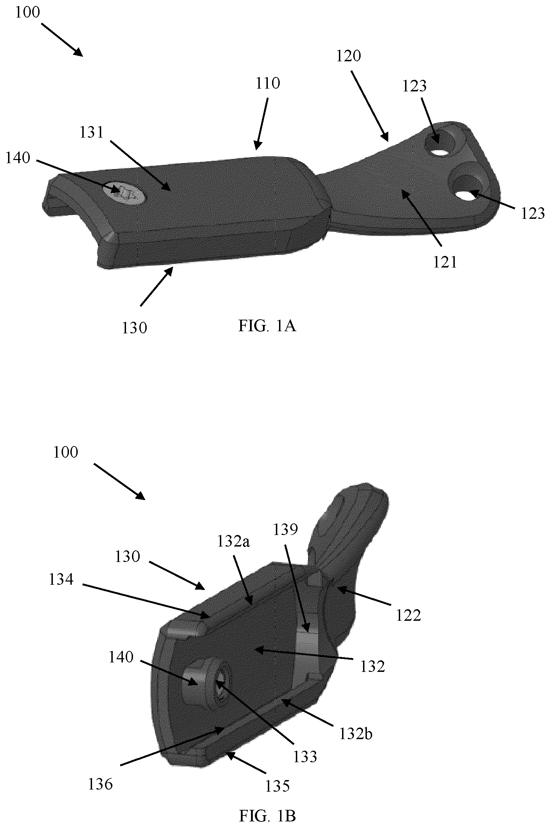

Referring to FIGS. 1A-4E, an expandable bone plate system 10 is shown that includes an extension plate 100 and a bone plate 200. Bone plate system 10 is designed to be fixed to a bone of a patient by common fixation elements such as traditional bone screws, prophylactic nails or combinations of any common fixation elements. Bone plate system 10 is a combination bone plates and prophylactic screws used for stabilization of the femoral neck and is used for the treatment of long bone fractures in a patient, as described in greater detail below. While bone plate system 10 is specifically described to treat fractures in the metaphyseal region of a femur, bone plate system 10 may be designed and used for treatment of fractures with any other long bone, such as the tibia, fibula, humerus, radius, or ulna. In use, extension plate 100 allows for length adaption and optimal fit of bone plate system 10 for prophylactic stabilization or fixation along a vastus ridge of the femoral neck in the metaphyseal region of the femur.

As best shown in FIGS. 1A-1B, extension plate 100 includes a body 110 extending across the length of extension plate 100. Body 110 includes an anchoring portion 120 and a connection portion 130. As described in greater detail below, extension plate 100 is configured to be fixed to bone plate 200 and positioned against the femoral neck in the metaphyseal region of the femur.

Anchoring portion 120, as best shown in FIGS. 1A-1B, is designed to be attachable to the bone at a distal end of extension plate 100 and bone plate system 10. Anchoring portion 120 includes a top surface 121 configured to face away from the bone, and a bottom surface 122 configured to face towards the bone. Bottom surface 122 may be curved to match the contours of the bone. Anchoring portion 120 also includes apertures 123 extending from top surface 121 to the bottom surface 122 through the anchoring portion 120. Apertures 123 may be designed to receive prophylactic screws for fixation within the femoral neck of the bone of the patient. In the embodiment shown in FIGS. 1A-1B, anchoring portion 120 includes a first and second aperture 123. However, in other embodiments, anchoring portion 120 may include only a single aperture 123 for receiving only a single prophylactic screw, or a plurality of apertures 123 for receiving a plurality of respective prophylactic screws, as needed for the desired prophylactic stabilization. Additionally, aperture 123 may be designed to receive traditional locking or non-locking bone screws.

Connection portion 130, as best shown in FIGS. 1A-1B, is designed to be proximal of anchoring portion 120 within extension plate 100 and along bone plate system 10. Connection portion 130 includes a top surface 131 configured to face away from the bone, and a bottom surface 132 configured to face towards the bone. Connection portion 130 is substantially rectangular in shape but may be designed in other shapes or configurations.

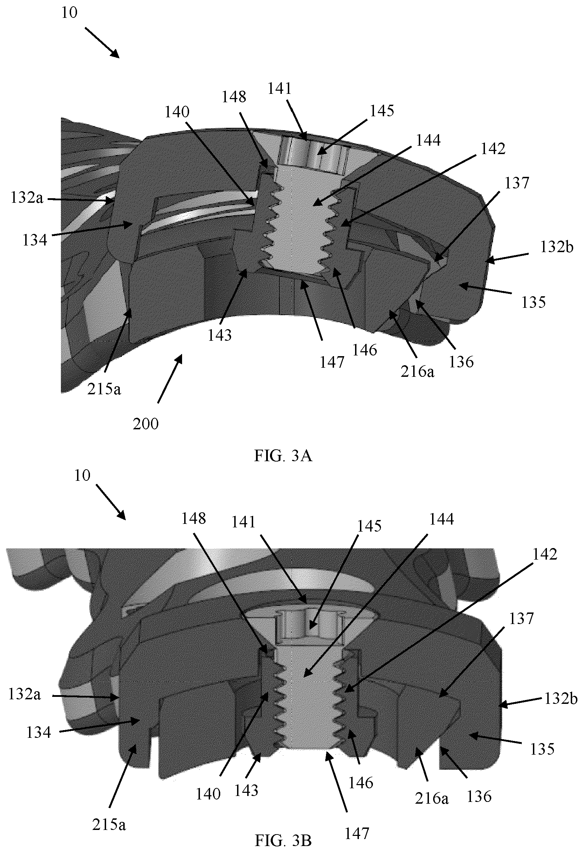

Bottom surface 132 of connection portion 130 has a first shelf 134 extending substantially perpendicular from a first side 132a of bottom surface 132, as best shown in FIGS. 3A-3B. Additionally, bottom surface 132 includes a second shelf 135, opposite first shelf 134, extending substantially perpendicular from a second side 132b of bottom surface 132. Second shelf 135 includes a rail 136 extending towards the first shelf 134. The extension of rail 136 towards the first shelf 134 defines an undercut 137 between rail 136 and bottom surface 132 of connection portion 130. Bottom surface 131 of connection portion 130 further includes an end shelf 139 extending substantially perpendicular from the bottom surface 130 and connecting ends of the first and second shelves 134, 135, as best shown in FIG. 1B. First and second shelves 134, 135 may be interchangeable such that first and second shelves 134, 135 are on opposite sides of connection portion 130 from the design shown in the embodiment.

Connection portion 130 further defines an aperture 133 extending from the top surface 131 to the bottom surface 132 of connection portion 130. Aperture 133 is disposed at proximal position of the connection portion 130 and the anchoring plate 120, but the aperture 133 may be positioned to extend through the connection portion 130 at any position of the connection portion 130. The top surface of connecting portion 130 extends along a substantially straight axis, and the top surface of anchoring portion 120 extends along a substantially curved axis. Because of this curvature or angle between connection portion 130 and anchoring plate 120, a central axis of aperture 123 in connection portion 120 and a central axis of aperture 133 in anchoring portion 130 can be non-parallel. This design allows the anchoring portion 120 to match the curvature of the bone as it extends from the connection 130. More specifically, when used with a femur, anchoring portion 120 extends along the curved vastus ridge of the femur to the femoral neck of the patient.

A locking element 140 is disposed through aperture 133 for locking extension plate 100 to bone plate 200, as best shown in FIGS. 3A-3B. Locking element 140 includes a proximal end 141, a distal end 143, and a middle portion 142 between the proximal end 141 and distal end 143. Proximal and distal ends 141, 143 each have a diameter that is larger than a minimum diameter of aperture 133. Middle portion 142, however, has a diameter that is smaller than the minimum diameter of the aperture 133.

Locking element 140 includes a locking screw 144 and a locking collar 146. The proximal end 141 of locking element 140 is a head 145 of locking screw 144. Further, the distal end 143 of the locking element 140 is locking collar 146. Locking screw 144 is configured to be threadedly inserted into a passage 147 of locking collar 146, such that rotation of the locking screw 144 with respect to locking collar 146 changes a length of locking element 140, the length measured between proximal end 141 and distal end 143. Head 145 of locking screw 144 includes a recess that is configured to accommodate an end of a tool that can be used to rotate locking screw 144 with respect to locking collar 146 in either direction. As discussed further below, locking element 140 is configured to fix extension plate 100 to bone plate 200 at a desired position relative to bone plate 200 and the bone of the patient.

Connection portion 130 further defines a recess 148 coaxial with aperture 133 and disposed within aperture 133. Recess 148 has a non-circular perimeter but may be designed with other shapes and configurations. Distal end 143 of locking collar 146 similarly is designed to have a non-circular out perimeter dimensioned to be disposed within recess 148 such that locking collar 146 is rotationally fixed with respect to recess 148.

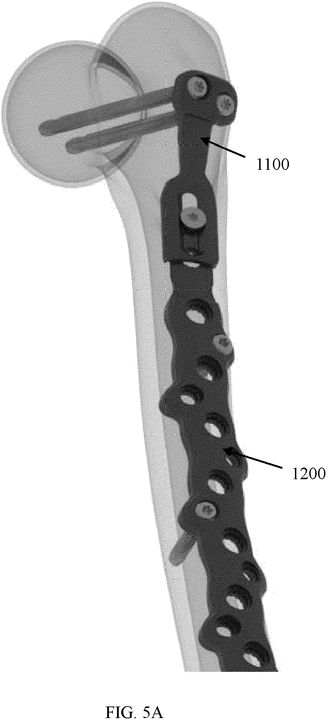

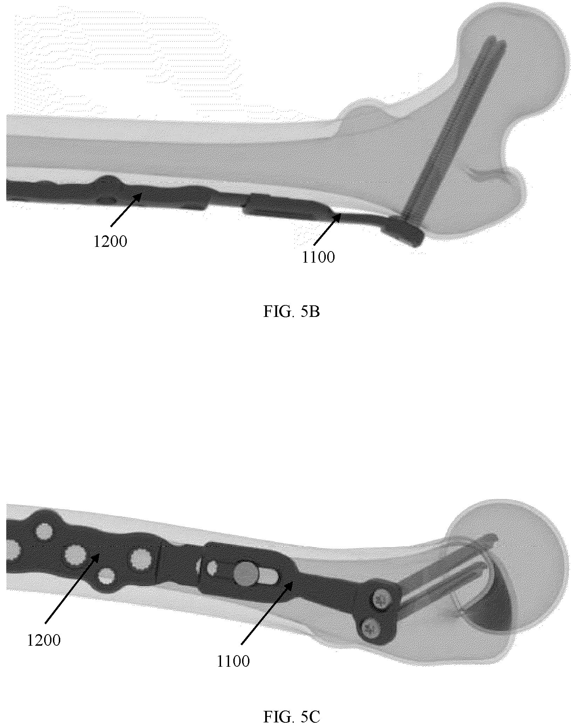

As previously mentioned, bone plate system 10 further includes bone plate 200. Bone plate 200 has an anchoring section 220 extending across a length of the bone. Anchoring section 220 includes a plurality of apertures 223 in which fasteners or locking screws can be disposed to anchor bone plate 200 to the bone. As best shown in FIGS. 5A-5B, a bone plate is fixed to the femur bone of the patient but may be fixed to other long bones as previously discussed.

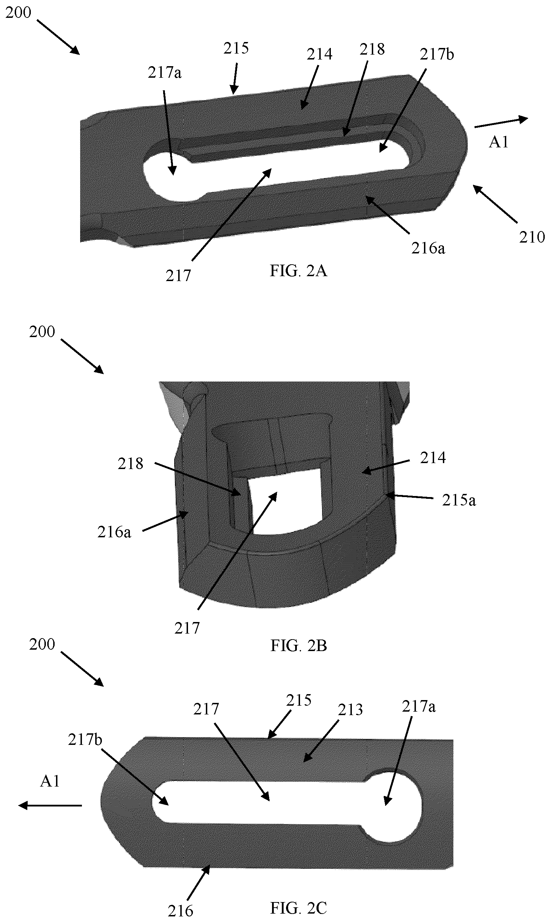

As best shown in FIGS. 2A-2C, a distal end 210 of bone plate 200 extends away from anchoring section 220 along a plate axis A1. In the embodiment, anchoring section 220 extends along the length of the femur of the patient such that the distal end 210 is positioned proximal to the femoral neck and femoral head of the patient. Distal end 210 includes a top surface 213 configured to face away from the bone, and a bottom surface 214 configured to face towards the bone. Distal end 210 includes a first edge 215 and a second edge 216 that each extend parallel to the plate axis A1. An outer surface 215a of first edge 215 extends from the top surface 213 to the bottom surface 214 along a first edge axis, and an outer surface 216a of second edge 216 extends from the top surface 213 to the bottom surface 214 along a second edge axis. Outer surface 216a of second edge 216 is angled respective to outer surface 215a of first edge 215, such that outer surface 216a of second edge 216 extends from top surface 213 to bottom surface 214 towards first edge 215. Therefore, as best shown in FIG. 2B, outer surface 215a extends substantially perpendicular to the plate axis A1. Outer surface 216a, however, is slanted along an angle forming a slant or ramped surface of second edge 216 that is not parallel or perpendicular to outer surface 215a.

Distal end 210 of bone plate 200 defines an opening 217 extending parallel to plate axis A1 in between first and second edges 215, 216. A proximal portion 217a of opening 217 is substantially circular in shape. Extending from proximal portion is a distal portion 217b of opening 217, and the distal portion 217b is substantially obround in shape. The width of the circular shape of proximal portion 217a in a direction perpendicular to axis A1 is greater than a width of the obround shape of distal portion 217b in a direction perpendicular to axis A1. Opening 217, however, may be modified to accommodate different shapes in either proximal and/or distal portions 217a, 217b. Distal portion 217b of opening 217 includes a rim 218 extending inwardly towards a center of the opening. Rim 218 extends along an entire inner surface of distal portion 217b of opening 217.

In a fully assembled state, FIGS. 5A-5C show an extension plate 1100 and a bone plate 1200 fixed into the bone of the patient. This embodiment indicates the final positioning of system 10 although with differently configured components. Namely, extension plate 1100 includes an elongate opening through which a fixation screw is inserted into a circular opening in plate 1200 beneath. This enables extension plate 1100 to be moved to a different position with respect to implanted bone plate 1200 before the fixation screw is tightened to secure extension plate 1100 with respect to bone plate 1200.

A method for extending a length of a bone plate system along a bone is performed using bone plate system 10. While bone plate system 10 may be used for treatment of a fracture of the femoral neck in the metaphyseal region of the femur, other long bones for which for which treatment of a fracture is required may also utilize bone plate system 10. The method for using bone plate system 10 may be for prophylactic fixation or stabilization of the femoral neck of a femur bone of a patient. While bone plate system 10 is mainly used in the description below, the other aforementioned systems may also be used by the same or similar methods when accounting for the variations in structure described above.

Anchoring section 220 of bone plate 200 is anchored onto the bone of the patient. Anchoring section 220 is designed to be fixed to the bone by common fixation elements such as bone screws, locking screws, or the like. Anchoring section 220 is fixed to the bone along the plate axis A1 which extends substantially along the length of the bone. As previously described and shown in the embodiment, the bone is a femur of the patent and the anchoring section 220 extends along the plate axis towards the femoral neck and femoral head of the patient.

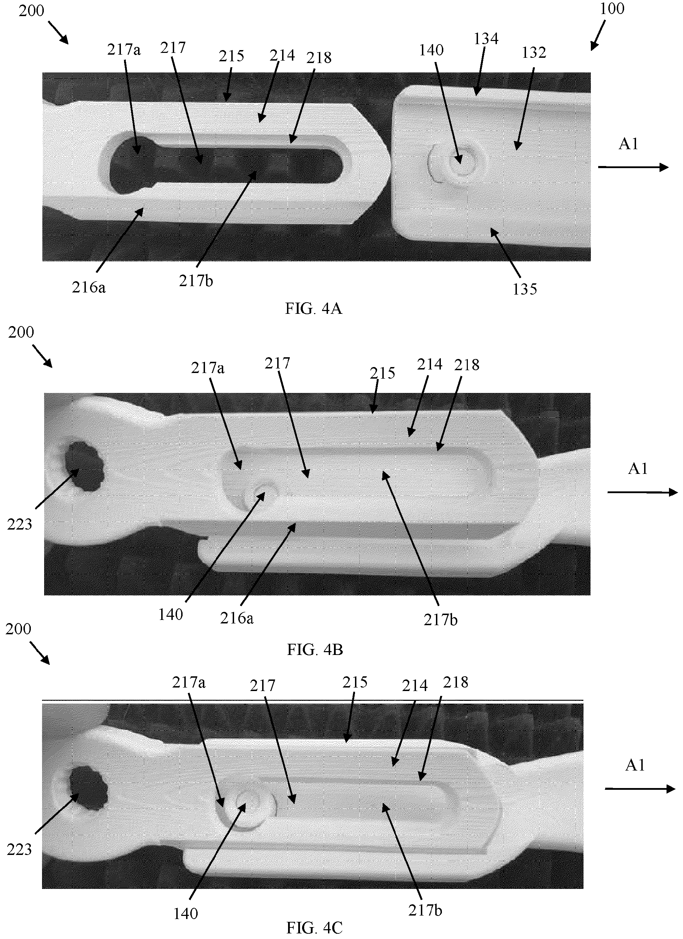

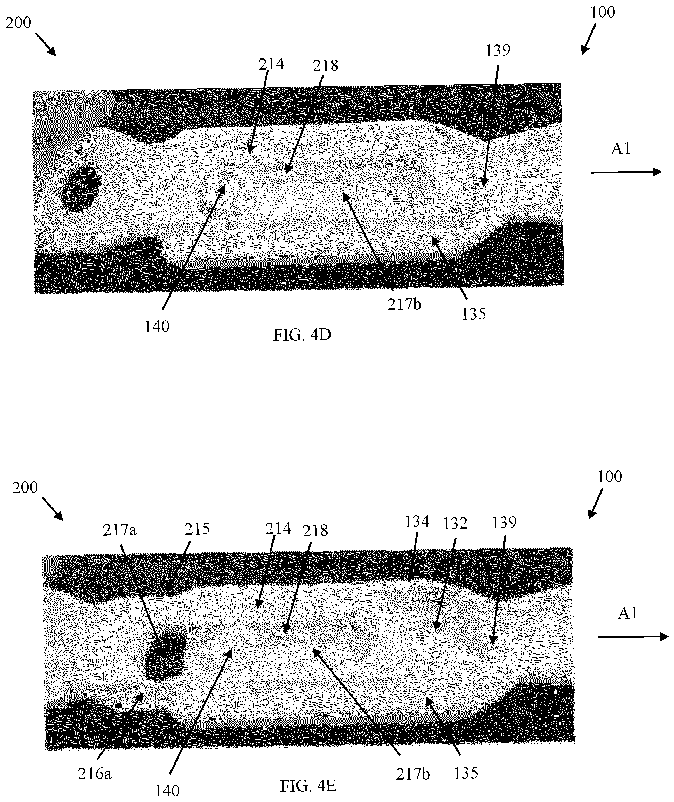

The connection portion 130 of extension plate 100 is fixed to a distal end 210 of bone plate 200. As best shown in FIGS. 4A-4E, connection portion 130 is first is titled at an angle about plate axis A1 to engage the distal end 210. As best shown in FIG. 3A-3B, connection portion 130 is positioned such that outer surface 216a of second edge 216 is disposed within undercut 137. Once positioned, connection portion 130 is rotated though another angle around the plate axis A1 until the connection portion 130 is properly secured to distal end 210, as best shown in FIG. 3B. A benefit of this titling motion to fix the connection portion 130 to the distal end is that it is not necessary to provide an increased incision length into the patient to accommodate the extension plate 100. For example, the same incision used for fixing the bone plate 200 to the bone may be substantially used for fixing the extension plate into the patient and onto the bone plate 100 and the bone. While this step of fixing connection portion 130 to distal end 210 of bone plate 200 tends to be performed after anchoring the anchoring section 220 to the bone, the steps can be performed in either order.

After fixing connection portion 130 to distal end 210 as described, extension plate 100 is slid along plate axis A1 until reaching a desired position along the bone as required for the specific anatomical structure of the patient. Extension plate 100 is configured to slide in first direction along plate axis A1 away from bone plate 200 and/or in a second direction along plate axis A1 toward bone plate 200. For example, the sliding movement of extension plate 100 allows the anchoring portion 120 to be positioned in various locations against the femoral neck of the patient. This provides additional modularity for the bone plate system 10 by allowing intraoperative design and modification the extension plate 100 positioning relative to the bone plate 200. Connection portion 130 of extension plate 100 is configured to be secured to distal end 210 of bone plate 200, such that anchoring portion 120 of extension plate 100 can be secured to the bone so that the bone plate system 10 has a length longer than a length of the bone plate 200 alone. The sliding step can occur before anchoring any components to the bone plate.

After sliding the extension plate 100 to the desired position along the bone, and with bone plate 200 preferably anchored to the bone, connection portion 130 is locked and secured to distal end 210. The locking step includes securing locking screw 144 extending through aperture 133 of connection portion through opening 217 of distal end 210. Proximal portion 217a of opening 217 is configured to have a larger diameter than distal portion 217b of opening 217 to initially receive locking element 140 prior to the sliding step. Only after locking element 140 is fully disposed through proximal portion 217a and the extension plate 100 is slid to the desired position along the bone, can locking element 140 be locked to any location, including distal portion 217b, of opening 217. Locking element 140 is secured to distal portion 217b of opening 217 as locking screw 144 is sufficiently rotated such locking collar 146 engages rim 218 of distal portion 217b.

After connection portion 130 is locked to distal end 210, anchoring portion 120 of extension plate 100 is secured to the bone. Extension plate 100 may be anchored to the bone by moving anchoring portion 120 in any direction away from plate axis A1. For example, anchoring portion 120 may be rotated about an axis parallel to plate axis A1, or anchoring portion 120 may be rotated about an axis traverse to plate axis A1. For example, this flexibility allows anchoring portion to be positioned along various points of the femoral neck. In embodiment where extension plate 100 is rigid, the sliding of extension plate 100 with respect to bone plate 200 is enough to align the properly angled extension plate 100 against the surface of the femur adjacent the femoral neck so that a fixation screw can be anchored into the femoral neck through extension plate 100.

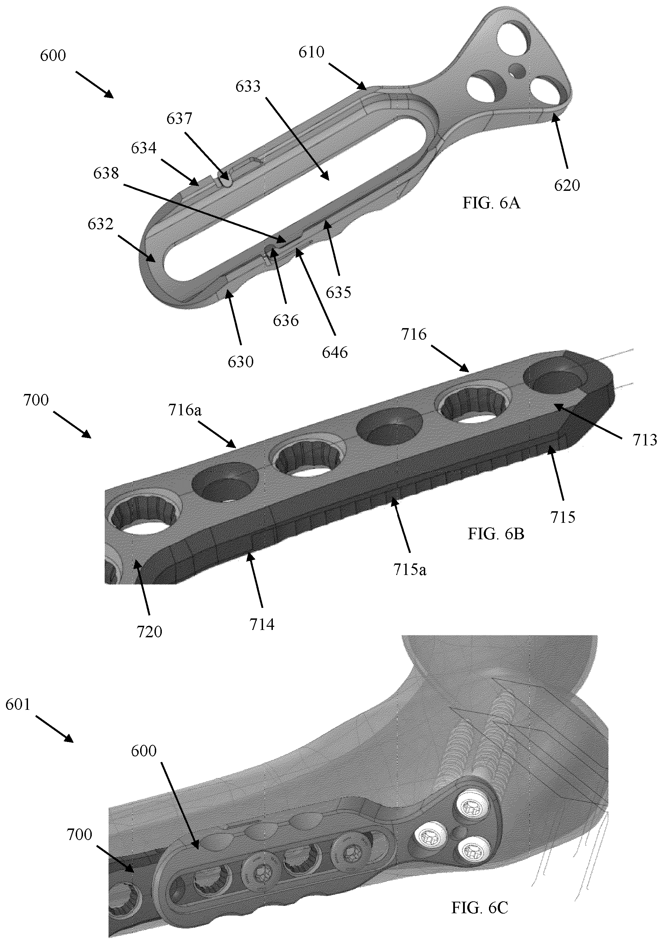

In another embodiment shown in FIGS. 6A-6C, a bone plate system 601 includes an extension plate 600 and a bone plate 700 that utilize a snap-on connection with a ratcheting mechanism. Many of the features of system 601 are similar to those of system 10, and the differences will therefore be noted below.

Extension plate 600 includes a body 610 having an anchoring portion 620 and a connection portion 630. Extending from bottom surface 632 of connection portion 630 are a first shelf 634 and a second shelf 635. Second shelf 635 includes a rail 636 that takes the form of a protrusion that extends toward first shelf 634. Protrusion 636 is disposed on the end of an arm 646 that is separated from the rest of second shelf 635 such that it acts as a cantilever. Protrusion 636 is relatively short and small compared to second shelf 635, such that it has a length shorter than a length of second shelf 635 when measured along the same longitudinal axis of extension plate 600. Arm 646 is defined by a cutout in second shelf 635 and is attached to the rest of second shelf 635 at one end, with protrusion 636 disposed at another end of arm 646. In this way, arm 646 is able to flex in different directions, including towards and away from first shelf 634 by pivoting about its anchor point with the rest of second shelf 635. Protrusion 636 juts out toward first shelf 635 such that an undercut 638 is formed between protrusion 636 and bottom surface 632.

First shelf 635 also includes a rail 637 that takes the form of a protrusion in the same way and with the same structure as described in connection with protrusion 636. In some embodiments, connection portion 630 is symmetrical so that first and second shelves 634, 635 are mirror images of one another. An aperture 633 is disposed in connection portion 630 between first and second shelves 634, 635.

Distal end 710 of bone plate 700 includes first and second edges 715, 716. An outer surface 715a of first edge 715 extends from the top surface 713 to the bottom surface 714 along a first edge axis. The same is true for an outer surface 716a of second edge 716. Both of surfaces 715a and 716a include ridges or scallops so that there are a series of depressions one after another in which protrusions 636 and 637 can be disposed. Both of surfaces 715a and 716a are beneath overhanging ledges that can be disposed between protrusions 636 and 637 and bottom surface 632 when extension plate 600 is attached with bone plate 700.

A method of using bone plate system 601 is similar to the method described above aside from the process of fixing the connection portion 630 of the extension plate 600 to the distal end 710 of the bone plate 700. With system 601, the step of fixing occurs by sliding the extension plate 600 over the distal end 710 and towards the bone plate 700 along the plate axis. During this movement, protrusions 636 and 637 pass over and ratchet along the ridges of surfaces 715a and 716a while also passing beneath the overhanging ledges of surfaces 715a and 716a, allowing the protrusions 636 and 637 to engage specific ridges to temporarily fix the extension plate 600 to the bone plate 700. This allows extension plate 600 to be put in one of many positions along distal end 710 before being anchored to the bone plate 700 and the underlying bone. In system 601, extension plate 600 includes an elongated aperture and distal end 710 of bone plate 700 includes a plurality of circular apertures, so that a fixation screw can be disposed in any one of the apertures on distal end 710 after passing through the elongated aperture of extension plate 600. This permits an attachment at many relative positions between extension plate 600 and bone plate 700 as extension plate 600 is clicked into place along bone plate 700.

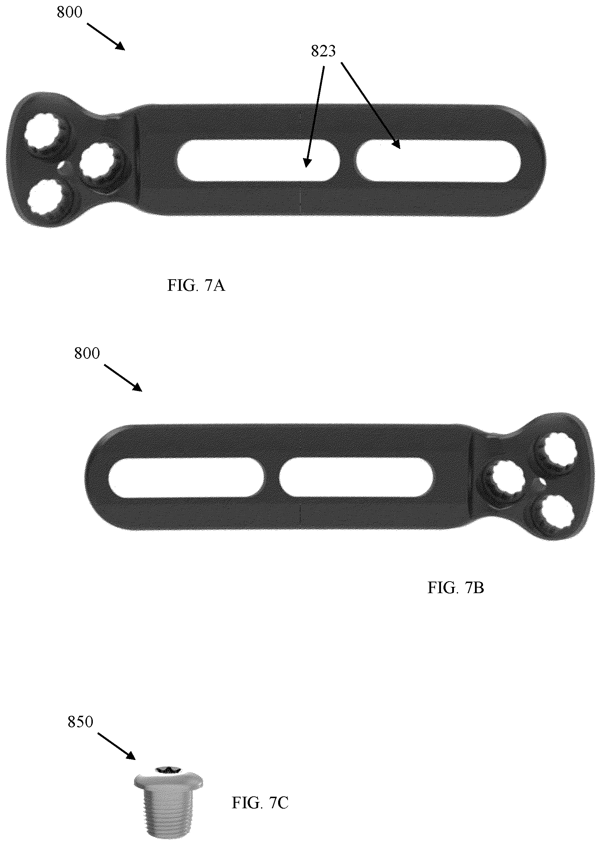

In another embodiment shown in FIGS. 7A-7C, an extension plate 800 is shown with two elongated apertures 823. A fixation screw 850 that can be disposed within one of the elongated apertures 823 to secure extension plate 800 to a bone plate is also shown.

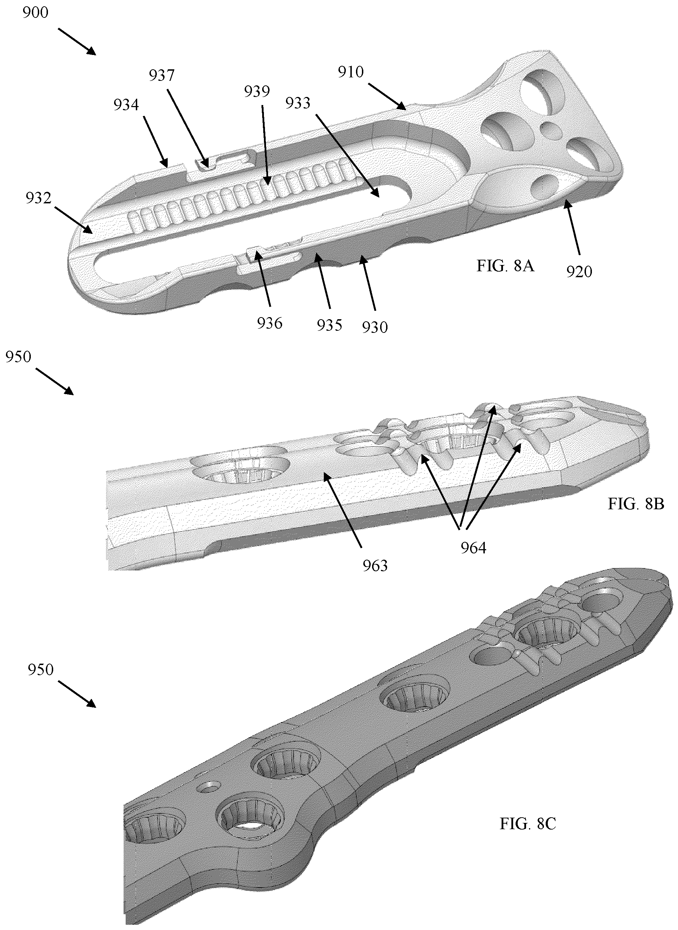

In another embodiment shown in FIGS. 8A-8C, a bone plate system includes an extension plate 900 and a bone plate 950 that also utilize a snap-on connection with a ratcheting mechanism. Many of the features of this system are similar to those of system 601, with only the differences noted below.

Extension plate 900 includes a body 910 having an anchoring portion 920 and a connection portion 930. Plate 900 also includes a first shelf 934 with a rail 937, and a second shelf 935 with a rail 936 as described in connection with system 601. A bottom surface 932 of extension plate 900 includes a series of ridges 939 which are in a linear arrangement parallel to the axis along which extension plate 900 is slid over bone plate 950. Ridges 939 can be a series of recesses or grooves in the material of extension plate 900 such that ridges are formed between the adjacent recesses or grooves. Ridges 939 can be placed adjacent first shelf 934 and/or adjacent second shelf 936 such they are on either or both sides of aperture 933.

The distal end of bone plate 950 includes a top surface 963 having one or more detents 964 protruding upward and away from top surface 963 that are configured to mate with ridges 939.

A method of using the bone plate system is similar to the method described above regarding system 601. The extension plate 900 is slid over and towards the bone plate 950 along the plate axis. During this movement, the ridges 939 pass over and ratchet along the detent(s) 934. This allows extension plate 900 to be put in one of many positions along the distal end of bone plate 950 before being anchored to the bone plate 950 and the underlying bone. This permits an attachment at many relative positions between extension plate 900 and bone plate 950.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. An extension plate attachable to a bone plate and a bone, the extension plate comprising:
a body having a connection portion connectable to a bone plate and an anchoring portion attachable to a bone;
the connection portion having a top surface configured to face away from a bone and a bottom surface configured to face toward the bone;
the bottom surface of the connection portion having a first shelf extending substantially perpendicular from the bottom surface at a first side of the bottom surface and a second shelf extending substantially perpendicular from the bottom surface at a second side of the bottom surface;
wherein the second shelf includes a rail extending toward the first shelf, such that the second shelf defines an undercut between the rail and the bottom surface.

2. The extension plate of claim 1, wherein the connection portion defines an aperture extending from the top surface to the bottom surface between the first and second sides in which a locking element can be disposed.

3. The extension plate of any of claims 1-2, wherein the anchoring portion has a top surface configured to face away from the bone and a bottom surface configured to face toward the bone, and an aperture extending from the top surface of the anchoring portion to the bottom surface of the anchoring portion in which a fastener can be disposed.

4. The extension plate of claim 3, wherein a central axis of the aperture in the connection portion and a central axis of the aperture in the anchoring portion are non-parallel.

5. The extension plate of any of claim 1-4, wherein along an axis extending from the connecting portion to the anchoring portion, the top surface of the connecting portion extends along a substantially straight axis, and the top surface of the anchoring portion extends along a substantially curved axis.

6. The extension plate of any of claims 1-5, wherein the bottom surface of the connection portion further includes an end shelf extending substantially perpendicular from the bottom surface and connecting ends of the first and second shelves.

7. The extension plate of any of claims 1-6, wherein the bottom surface of the connection portion defines ridges, and the ridges are configured to mate with a detent on a bone plate.

8. The extension plate of any of claims 1-7, wherein the rail is a protrusion disposed at the end of an arm.

9. The extension plate of claim 8, wherein the arm is a cantilever beam defined by a cutout in the second shelf, and the arm is configured to flex toward and away from the first shelf.

10. The extension plate of any of claims 8-9, wherein the first shelf includes a rail extending toward the second shelf, such that the first shelf defines an undercut between the rail of the first shelf and the bottom surface, wherein the rail of the first shelf is a protrusion disposed at the end of an arm, and wherein the arm of the first shelf is a cantilever beam defined by a cutout in the first shelf.

11. The extension plate of claim 10, wherein the first shelf is a mirror image of the second shelf.

12. The extension plate of any of claims 1-11, further comprising a locking screw.

13. A bone plate system comprising:
a bone plate having an anchoring section and a distal end extending from the anchoring section, wherein the anchoring section defines an aperture in which a fastener can be disposed to anchor the bone plate to a bone; and
the extension plate of any of claims 1-12,
wherein the connection portion of the extension plate is configured to be secured to the distal end of the bone plate, such that the anchoring portion of the extension plate can be secured to the bone so that the bone plate system has a length longer than a length of the bone plate.

14. The bone plate system of claim 13, wherein the distal end of the bone plate extends away from the anchoring section of the bone plate along a plate axis, the distal end of the bone plate having first and second side edges extending parallel to the plate axis, the distal end of the bone plate having a top surface configured to face away from a bone and a bottom surface configured to face toward the bone,
wherein an outer surface of the first edge extends from the bottom surface to a ledge at the top surface along a first edge axis, and an outer surface of the second edge extends from the bottom surface to a ledge at the top surface along a second edge axis.

15. The bone plate system of claim 14, wherein the outer surfaces of the first and second edges each include ridges, and the ridges are configured to engage the protrusion disposed at the end of the arm.
